# EUROPÄISCHE PATENTANMELDUNG

(11) **EP 0 798 040 A1**
(43) Veröffentlichungstag der Anmeldung: **01.10.1997**
(21) Anmeldenummer: 97103700.7
(22) Anmeldetag: 06.03.1997
(51) Int. Cl.: B01J 27/16, C07C 29/04

(54) **Phosphorsäurekatalysator und seine Verwendung**

(30) Priorität: 29.03.1996 DE 19612783
(71) Anmelder: DEGUSSA AG, 60311 Frankfurt (DE)
(72) Erfinder: Lansink-Rotgerink, Hermanus, Dr., 63864 Glattbach (DE); Höcker, Harald, Dr., 63834 Sulzbach (DE); Wieland, Stefan, Dr., 63069 Offenbach (DE); Seebald, Steffen, Dr., 63538 Grosskrotzenburg (DE); Riedemann, Heike, 63776 Mömbris (DE)

(57) **Zusammenfassung**

Die vorliegende Erfindung betrifft einen Phosphorsäurekatalysator auf einem geformten, anorganischen Träger und seine Verwendung für die Hydratisierung von Olefinen. Der Katalysatorträger besteht zu mindestens 90 Gew.-% aus Titan- und/oder Zirkondioxid und weist eine hervorragende Alterungsstabilität unter den hydrothermalen Bedingungen bei der Hydratisierung auf.

## Beschreibung

Die Erfindung betrifft einen Phosphorsäurekatalysator und seine Verwendung für katalytische Reaktionen unter hydrothermalen Bedingungen. Hydrothermale Bedingungen liegen bei chemischen Umsetzungen in wäßrigen Systemen vor, wenn bei Temperaturen oberhalb des Siedepunktes von Wasser und bei Drucken oberhalb von Normaldruck gearbeitet wird.

Eine typische Umsetzung unter hydrothermalen Bedingungen ist die Hydratisierung von Olefinen zu den entsprechenden Alkoholen in Gegenwart von Phosphorsäure als Katalysator beziehungsweise Aktivkomponente auf einem Siliziumdioxid enthaltenden geformten Katalysatorträger.

Ein solches Verfahren wird zum Beispiel in der EP 0 578 441 A2 beschrieben. Nach diesem Verfahren wird Wasser und Ethylen bei Temperaturen zwischen 225 und 280°C und Drucken zwischen 20 und 240 bar zu Ethanol umgesetzt. Dabei wird ein Wasser/Ethylen-Molverhältnis im Bereich von 0,15 bis 0,5 angewendet. Die Katalysatorbelastung, gemessen in Gramm Wasser/Ethylen-Gemisch pro Minute und Millimeter Katalysator, kann im Bereich von 0,01 bis 0,1 g/(min × ml) gewählt werden. Als Nebenprodukt wird bei dieser Reaktion Diethylether gebildet.

Die Herstellung von Isopropanol durch Hydratisierung von Propylen erfolgt unter ähnlichen Bedingungen wie oben angegeben, jedoch bei etwas verringerter Temperatur im Bereich zwischen 180 und 225°C. Als Nebenprodukt entsteht bei dieser Reaktion n-Propanol.

Als Katalysatorträger für die Aktivkomponente Phosphorsäure verwendet die EP 0 578 441 A2 Pellets aus synthetischem Siliziumdioxid mit hoher Bruchfestigkeit, hoher Porosität und geringen metallischen Verunreinigungen. Die Poren des Trägers dienen zur Aufnahme der Aktivkomponente. Das Porenvolumen ist daher bevorzugt größer als 0,8 ml/g. Der mittlere Porenradius vor dem Einsatz im Hydratisierungsprozeß liegt im Bereich zwischen 1 und 50 nm.

Zur Erzielung einer optimalen Leistung bei der Hydratisierung wird von der EP 0 578 441 A2 ein Gehalt des Trägers an Siliziumdioxid von wenigstens 99 Gew.-% vorgeschrieben mit Verunreinigungen unter 1 Gew.-%, bevorzugt unter 0,3 Gew.-%.

Katalysatoren unterliegen im Betrieb einer Alterung, die sich durch eine Verringerung der Aktivität und/oder Selektivität bemerkbar macht. Die Desaktivierung beruht häufig auf einer durch hohe Temperaturen verursachten Verringerung der spezifischen Oberfläche des Trägers.

Die spezifische Oberfläche des Trägers ist eng mit seiner Porenstruktur verbunden. Darüber hinaus weisen hochoberflächige Feststoffe meist eine vollständig amorphe oder überwiegend amorphe Struktur auf, welche das Bestreben hat, unter Kristallitwachstum und Verringerung der spezifischen Oberfläche in einen thermodynamisch stabilen Zustand überzugehen.

Es hat sich gezeigt, daß Siliziumdioxid enthaltende Katalysatorträger einer solchen Alterung unterliegen. Hydrothermale Bedingungen beschleunigen die Alterung.

Dies gilt auch für die in der EP 0 393 356 beschriebenen Katalysatorträger auf Basis von pyrogen hergestelltem Siliziumdioxid. Durch Zusammenwachsen kleinerer Poren zu größeren Poren verringert sich die spezifische Oberfläche.

Das Porenvolumen ändert sich dabei anfänglich kaum. Diese Alterung tritt ein, obwohl das pyrogene Siliziumdioxid, aus denen die Träger bestehen, eine hervorragende Temperaturbeständigkeit aufweist. Beim Erhitzen auf Temperaturen bis zu 1000°C für die Dauer von 7 Tagen ändert pyrogenes Siliziumdioxid nach Untersuchungen mit einem Rasterelektronenmikroskop seine Morphologie nicht (Schriftenreihe Pigmente Nr. 11: Grundlagen von AEROSIL®; Firmenschrift der DEGUSSA, 5. Auflage, Juni 1993, Seite 20).

Aufgabe der vorliegenden Erfindung ist es, einen Phosphorsäurekatalysator auf einem Katalysatorträger anzugeben, welcher bei Verwendung unter hydrothermalen Bedingungen eine verbesserte Alterungsstabilität aufweist.

Diese Aufgabe wird durch einen Phosphorsäurekatalysator gelöst, welcher auf einem geformten, anorganischen Träger 1 bis 50 Gew.-% H₃PO₄ bezogen auf das Gesamtgewicht des Katalysators enthält. Er ist dadurch gekennzeichnet, daß der Träger zu mindestens 90 Gew.-%, bezogen auf das Gesamtgewicht des reinen Trägers, aus Titan- und/oder Zirkondioxid besteht.

Als Titan- und Zirkondioxid eignen sich Materialien wie sie in der Katalyse üblicherweise eingesetzt werden. Es kann sich dabei um naßchemisch nach dem Sulfat- oder Chloridprozeß gewonnene Materialien oder durch Flammenhydrolyse hergestellte, sogenannte pyrogene Titan- oder Zirkonoxide handeln. Sie bestehen zu wenigstens 90 Gew.-% aus Titanbeziehungsweise Zirkondioxid. Ihr Glühverlust beträgt in der Regel weniger als 5 Gew.-%. Sie können je nach Herkunft der Ausgangsmaterialien zum Beispiel geringe Anteile von Siliziumdioxid, Aluminiumoxid, Eisenoxid, Alkalioxide und Phosphorpentoxid enthalten. Je nach Herstellungsprozeß weisen sie außerdem einen Sulfat- oder Chloridgehalt von 1 bis 2 Gew.-% auf. Im Falle von Titandioxid sind Materialien mit Anatas oder Rutilstruktur geeignet. Bevorzugt werden wegen ihrer hohen Porosität und hohen spezifischen Oberfläche Materialien verwendet, die ausschließlich oder überwiegend (mehr als 80 %) die Struktur des Anatas aufweisen.

Die genannten Materialien werden in an sich bekannter Weise zu Katalysatorformkörpern verarbeitet. Bei den Katalysatorformkörpern kann es sich um Extrudate, Granulate oder Tabletten handeln. In der DE 40 12 479 A1, der EP 0 394 677 B1 und der EP 0 389 041 A1 werden geeignete Verfahren zur Herstellung solcher Formkörper aus den pulverförmigen Materialien beschrieben.

Zur Herstellung der erfindungsgemäßen Katalysatoren werden die Formkörper mit Phosphorsäure als Aktivkomponente belegt. Hierzu werden die Trägerteilchen beziehungsweise Formkörper in eine wäßrige Lösung von Phosphorsäure getaucht und mit dieser getränkt, wodurch sich das Porenvolumen der Trägerteilchen mit der Lösung der Phosphorsäure füllt. Zur Anwendung kommen hierbei Phosphorsäurelösungen mit 15 bis 85 Gew.-% Phosphorsäure bezogen auf das Gesamtgewicht der Lösung. Abhängig vom jeweiligen Porenvolumen der Trägerteilchen können sie auf diese Weise mit 1 bis 50 Gew.-% H₃PO₄ bezogen auf das Gesamtgewicht des fertigen Katalysators belegt werden.

Die so hergestellten Katalysatoren werden für die Hydratisierung von Olefinen verwendet. Ein Hauptanwendungsgebiet der Hydratisierung von Olefinen ist die Hydratisierung von Ethylen zur Herstellung von Ethanol und Diethylether sowie die Hydratisierung von Propylen zur Herstellung von Isopropanol. Es werden dabei die aus dem Stand der Technik bekannten Reaktionsbedingungen angewendet.

Die Katalysatorkörper werden hierzu in Form einer Schüttung in einen Hochdruckreaktor eingebracht und von den Reaktanden umströmt. Um eine ausreichende Menge der Aktivkomponente Phosphorsäure pro Volumen des Reaktors zur Verfügung zu stellen, sollte das Porenvolumen der Formkörper 100 bis 500 cm³ pro Liter Schüttvolumen betragen. Bei einer Schüttdichte der Formkörper von beispielsweise 1000 g/l sollte daher das auf ihre Masse bezogene, spezifische Porenvolumen der Formkörper im Bereich zwischen 0,1 und 0,5 cm³/g liegen.

Die Anmessungen der Katalysatorkörper beeinflussen zum einen den Druckverlust im Reaktor und zum anderen die Zugänglichkkeit der in den Poren befindlichen Phosphorsäure für die Reaktanden. Bei zu kleinen Anmessungen wird der Druckverlust zu hoch, bei zu großen Abmessungen verringert sich die Zugänglichkeit der Poren im Innern der Katalysatorkörper. Als geeignet haben sich Abmessungen im Bereich zwischen 1 und 10 mm erwiesen. Bevorzugt werden Formkörper mit Abmessungen zwischen 2 und 5 mm eingesetzt.

Zur Untersuchung der Alterungsstabilität der erfindungsgemäßen Katalysatoren und von Vergleichskatalysatoren aus dem Stand der Technik wurde die Porenstruktur der frischen, noch nicht mit Phosphorsäure getränkten Träger und der gealterten Katalysatoren, d. h. nach Tränken der Träger mit Phosphorsäure und hydrothermaler Alterung, ermittelt. Die Bestimmung der Porenstruktur erfolgte mit Hilfe der Quecksilberporosimetrie. Die Beladung der Katalysatorkörper mit Phosphorsäure hat auf die mit der Quecksilberporosimetrie bestimmbare Porenstruktur einen vernachlässigbaren Einfluß. Die Beladung mit Phosphorsäure vermindert allerdings das meßbare Gesamtporenvolumen und muß daher bei der Berechnung des tatsächlichen Porenvolumens berücksichtigt werden.

Es zeigen
- Figur 1:: Porenstruktur eines frischen Trägers aus pyrogener Kieselsäure und nach Belegen des Trägers mit Phosphorsäure und hydrothermaler Alterung
- Figur 2:: Porenstruktur eines frischen Trägers aus natürlicher Kieselsäure und nach Belegen des Trägers mit Phosphorsäure und hydrothermaler Alterung
- Figur 3:: Porenstruktur eines frischen Trägers aus Titandioxid und nach Belegen des Trägers mit Phosphorsäure und hydrothermaler Alterung

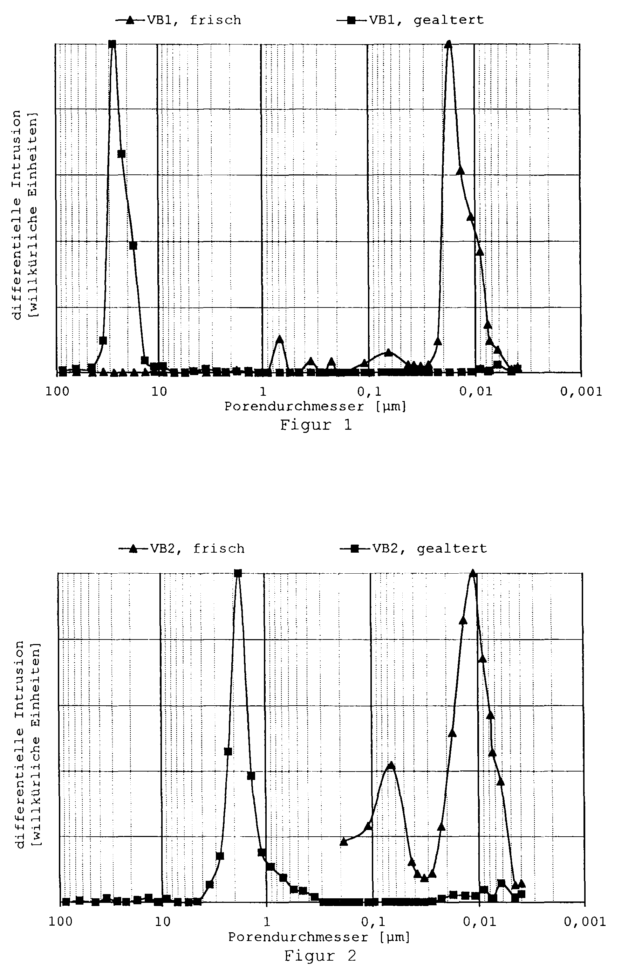

Die in den Figuren 1 bis 3 gezeigten Porenverteilungskurven geben die differentielle Eindringung (Intrusion) des Quecksilbers in Abhängigkeit vom Porendurchmesser wieder. Für die differentielle Intrusion wurden willkürliche Einheiten gewählt und die Kurven jeweils über den zur Verfügung stehenden Diagrammbereich gedehnt.

### Vergleichsbeispiel 1

Ein Katalysatorträger aus pyrogener Kieselsäure (AEROSIL^{®}-Träger 350 von Degussa; spezifische Oberfläche 180 m²/g; Schüttdichte 490 g/l; gesamtes Porenvolumen 0,8 cm³/g; Tabletten von 6 mm Durchmesser und 5,5 mm Höhe) wurde mit Phosphorsäure (60 Gew.-%)getränkt und 42 Stunden in einer Hochdruckanlage bei einem Wasserdampfdruck von 15 bar auf 350°C geheizt. Die Porenverteilung des frischen Trägers sowie des gealterten Katalysators wurde mit Hg-Porosimetrie bestimmt. Die gemessenen Porenverteilungskurven sind in Figur 1 grafisch dargestellt (VB1 frisch; VB1 gealtert).

Das Maximum der Porenverteilungskurve des frischen Trägers liegt bei Porendurchmessern zwischen 10 und 20 nm. Durch die hydrothermale Alterung unter Einwirkung der Phosphorsäure in den Poren verschiebt sich das Maximum zu Porendurchmessern zwischen 20 und 30 µm. Es hat also ein Porenwachstum um den Faktor 1000 stattgefunden. Das Gesamtporenvolumen wird jedoch durch das Porenwachstum kaum verändert. Es wachsen offensichtlich die kleinen Poren zu größeren zusammen.

### Vergleichsbeispiel 2

Ein Katalysatorträger auf Basis natürlicher Kieselsäure (spezifische Oberfläche 161 m²/g; Porenvolumen 0,67 cm³/g; Schüttdichte 550 g/l, Kugeln mit einem Durchmesser von 5 mm) wurde mit Phosphorsäure (60 Gew.-%)getränkt und 42 Stunden in einer Hochdruckanlage bei einem Wasserdampfdruck von 15 bar auf 350°C geheizt. Die Porenverteilung des frischen Trägers sowie des gealterten Katalysators wurde mit Hg-Porosimetrie bestimmt. Die gemessenen Porenverteilungskurven sind in Figur 2 grafisch dargestellt (VB2 frisch; VB2 gealtert).

Auch bei diesem Trägermaterial findet durch die hydrothermale Behandlung ein dramatisches Porenwachstum statt.

### Beispiel 1

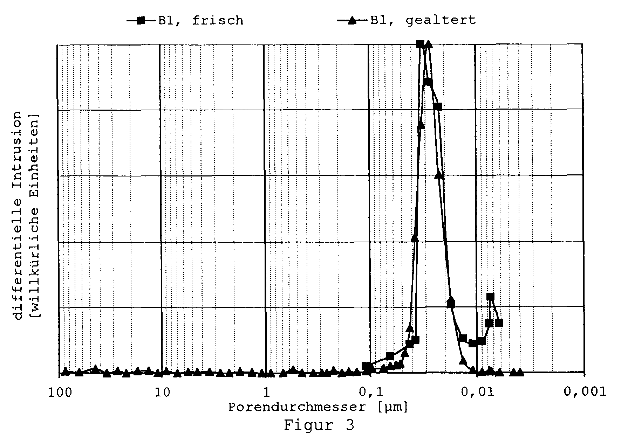

Ein Katalysatorträger aus Titandioxid (spezifische Oberfläche 165 m²/g; Porenvolumen 0,32 cm³/g; Schüttgewicht 900 g/l, Extrudate mit einem Durchmesser von 3 mm, Länge 2 - 4 mm; Kristallstruktur überwiegend Anatas; Gehalt an TiO₂ größer als 97 Gew.-%) wurde mit Phosphorsäure 60 Gew.-%)getränkt und 42 Stunden in einer Hochdruckanlage bei einem Wasserdampfdruck von 15 bar auf 350°C geheizt. Die Porenverteilung des frischen Trägers sowie des gealterten Katalysators wurde mit Hg-Porosimetrie bestimmt. Die gemessenen Porenverteilungskurven sind in Figur 3 grafisch dargestellt (B1 frisch; B1 gealtert).

Die Porenstruktur dieses Katalysators ändert sich gegenüber dem frischen Trägermaterial durch die hydrothermale Alterung kaum. Diese Messungen belegen die sehr gute Langzeitstabilität der Phosphorsäurekatalysatoren auf Basis von Titandioxid enthaltenden Katalysatorträgern.

## Patentansprüche

1. Phosphorsäurekatalysator, welcher auf einem geformten, anorganischen Träger 1 bis 50 Gew.-% H₃PO₄ bezogen auf das Gesamtgewicht des Katalysators enthält,
**dadurch gekennzeichnet,**
daß der Träger zu mindestens 90 Gew.-% bezogen auf das Gewicht des reinen Trägers aus Titan- und/oder Zirkondioxid besteht.

2. Verwendung des Katalysators nach Anspruch 1 für die Hydratisierung von Olefinen.

3. Verwendung nach Anspruch 2 für die Hydratisierung von Ethylen zur Herstellung von Ethanol und Diethylether.

4. Verwendung nach Anspruch 2 für die Hydratisierung von Propylen zur Herstellung von Isopropanol.
